# EUROPEAN PATENT APPLICATION

(11) **EP 4 574 812 A1**
(43) Date of publication of application: **25.06.2025**
(21) Application number: 24851037.2
(22) Date of filing: 07.08.2024
(51) Int. Cl.: C07C 231/02, C07C 233/47

(54) **METHOD FOR SYNTHESIZING HIGH-PURITY N-LAUROYL LYSINE**

(30) Priority: 09.08.2023 CN 202310995659
(71) Applicant: Changsha Puji Biotechnology Co., Ltd., Changsha, Hunan 410329 (CN)
(72) Inventor: LI, Jinwei, Changsha, Hunan 410329 (CN); YU, Mingqiang, Changsha, Hunan 410329 (CN); ZHU, Jiyou, Changsha, Hunan 410329 (CN); YANG, Wen, Changsha, Hunan 410329 (CN); WANG, Jinping, Changsha, Hunan 410329 (CN)
(74) Representative: Behr, Wolfgang
(86) International application number: PCT/CN2024/110364
(87) International publication number: WO 2025/031392

(57) **Abstract**

The present invention belongs to the technical field of fine synthesis, and discloses a method for synthesizing high-purity N^{ε}-lauroyl lysine. The method comprises: stirring raw materials including lysine and/or a lysine salt, an alkaline compound, water, a lower alcohol, and a water-insoluble solvent until uniform to form a two-phase microemulsion; and adding lauric acid and/or lauroyl halide dropwise to the two-phase microemulsion, reacting with stirring, and precipitating by neutralization with an acid, to obtain N^{ε}-lauroyl lysine. By constructing a two-phase reaction system, the method tactfully solves the problem regarding the reaction selectivity of α-amino and ε-amino and obtains high-purity N^{ε}-lauroyl lysine with a high yield. The reaction conditions are mild, and the separation and purification of the product are simple, so the method is suitable for use in industrial production.

## Description

### TECHNICAL FIELD

The present invention belongs to the technical field of chemical synthesis, and relates to a method for synthesizing N^{ε}-lauroyl lysine, and particularly to a two-phase method for synthesizing high-purity N^{ε}-lauroyl lysine with a high selectivity.

### BACKGROUND

N^{ε}-lauroyl lysine is an amino acid derivative, which has a poor solubility, is insoluble in water and most organic solvents, and is a functional hydrophobic powder insensitive to solvents. It has the advantages such as high lubricity, good skin-friendliness, anti-static performance, oxidation resistance, antibacterial performance and good biodegradability. It is often used as a surface modifier for powders, for hair and skin conditioning and makeup and cosmetic powder treatment; and also used as an additive for cosmetics, such as foundation lotion, eye shadow, rosy tint, and high-end cleansing products. Although there are many reports about the synthesis of N^{ε}-lauroyl lysine, most methods are not suitable for use in industrial production. Therefore the domestic demand mainly depends on the import from other countries.

### SUMMARY

### Technical problem

For the preparation of N^{ε}-lauroyl lysine, the most common chemical synthesis method is the condensation of lysine (lysine hydrochloride) with lauric acid or lauroyl chloride. Because lysine contains an α-amino and a ε-amino group in the structure, the reaction selectivity is poor during the condensation reaction (acylation reaction), and a large number of by-products N^{α}-lauroyl lysine and N,N-dilauroyl lysine tend to produced, causing the difficulty in separation and purification and a low yield of the target product N^{ε}-lauroyl lysine. Therefore, it is critical for this synthesis method to solve the problem of poor reaction selectivity, so as to realize the high-efficiency and high-selectivity preparation of N^{ε}-lauroyl lysine. To improve the reaction selectivity of α- and ε-amino groups, two common strategies are available. One is protection with a group, which can achieve accurate synthesis. Although accurate synthesis can be realized, there are many synthesis steps and the production cost is high. The other one is chelation with a metal ion. Metal residue in the product is easily caused, and a large amount of wastewater containing metal ions will be produced during post-treatment. For example, Patent Publication No. CN 102617390 B discloses a method for preparing N^{ε}-lauroyl lysine, in which lysine or a salt thereof and lauroyl chloride are used as raw materials, and a divalent metal salt (CaCl₂, ZnCl₂, MgCl₂, ZnSO₄, FeSO₄, and CuSO₄ etc.) is used for chelation protection. Lysine or its salt forms a chelate with a divalent metal ion to protect the α-amino group and carboxyl group, then the ε-amino group is condensed with lauroyl chloride, and finally the chelate structure is destroyed by acidolysis to obtain N^{ε}-lauroyl lysine.

### Technical solution

In view of the technical problem of poor reaction selectivity in the synthesis of N^{ε}-lauroyl lysine in the prior art, an object of the present invention is to provide a method for synthesizing high-purity N^{ε}-lauroyl lysine. In the method, a two-phase reaction system is used to control α-amino and carboxyl groups of lysine to gather in the aqueous phase, based on a synthesis idea of a "two-phase method". The ε-amino group is gathered in the oily phase and reacted with lauric acid or lauroyl halide, to greatly reduce the side reactions, and obtain N^{ε}-lauroyl lysine with a high selectivity.

In the present invention, the high-purity N^{ε}-lauroyl lysine is understood to have a purity of 92% or higher, preferably 95% or higher, and most preferably 97% or higher.

To achieve the above technical objects, the present invention provides a method for synthesizing high-purity N^{ε}-lauroyl lysine. The method includes the following steps:
1) stirring raw materials including lysine and/or a lysine salt, an alkaline compound, water, a lower alcohol, and a water-insoluble solvent until uniform to form a two-phase microemulsion; and
2) adding lauric acid and/or a lauric acid derivative dropwise to the two-phase microemulsion, reacting with stirring, and precipitating by neutralization with an acid, to obtain N^{ε}-lauroyl lysine.

In the technical solution of the present invention, a lysine salt is directly used as a raw material, or lysine is neutralized into a salt with an alkaline compound. The hydrophilicity of the carboxylate end is enhanced, and the hydrophobicity of the ε-amino end is relatively strong. Therefore, the lysine salt has both hydrophilicity and hydrophobicity. Based on this, a two-phase microemulsion reaction system is constructed. The α-amino group close to the carboxylate end is forcibly dissolved in the aqueous phase and protected from participation in the reaction. The ε-amino group is free at the interface between the oily phase and the aqueous phase or dissolved in the oily phase. Lauric acid or a lauric acid derivative added is mainly in the oily phase, and can be brought into contact and reacted with the amino group at the oil-water interface or in the oily phase, to synthesize N^{ε}-lauroyl lysine with a high selectivity.

In a preferred embodiment, the ratio of the total volume of the water and the lower alcohol to the volume the water-insoluble solvent is 1:1 to 1:5. The lower alcohol is an important component for constructing the two-phase microemulsion. The lower alcohol is miscible with water, but the solubility of the lower alcohol is relatively small in water-insoluble solvents such as petroleum ether, n-hexane, cyclohexane, toluene and dichloromethane. Therefore, when these water-insoluble solvents are mixed and dissolved in water and a lower alcohol at a preferred ratio, a stable two-phase microemulsion is advantageously constructed, which is mainly a water-in-oil microemulsion. If the proportion of the water-insoluble solvent is too high, the subsequent reaction will be inefficient and the cost will increase. If the proportion of the water-insoluble solvent is too low, it will be difficult to form a stable two-phase microemulsion, causing the decrease of the selectivity of the reaction and the difficulty in the treatment of the product. The ratio of the total volume of the water and the lower alcohol to the volume of the water-insoluble solvent is further preferably 0.8:1 to 1: 2.

In a preferred embodiment, the volume ratio of the water and the lower alcohol is (5-20): 40. The introduction of a proper amount of the lower alcohol is conducive to the formation of the stable two-phase microemulsion. Moreover, because of the compatibility of the lower alcohol in water-insoluble solvent, the lower alcohol can be used as a phase transfer agent in the reaction process, to bring the two phases into communication, thus promoting the lipophilic end of the lysine sodium salt as a raw material to enter the oily phase and improving the reaction efficiency. If the alcohol is not added, the water and the water-insoluble solvent cannot form a two-phase microemulsion, leading to the side reactions. For example, the lauroyl chloride is largely reacted to produce sodium laurate, a hydrolysis product of acyl chloride.

In a preferred embodiment, the lower alcohol is a C₁-C₄ alcohol, specifically including at least one of methanol, ethanol, isopropanol and n-butanol. Methanol or ethanol is further preferred.

In a preferred embodiment, the water-insoluble solvent includes at least one of petroleum ether, n-hexane, cyclohexane, toluene, and dichloromethane. The preferred water-insoluble solvents can well dissolve the lauric acid derivative, but are not miscible with water. Moreover, the ability of these water-insoluble solvent is worse than water.

**In** a preferred embodiment, the alkaline compound includes at least one of sodium hydroxide, potassium hydroxide, and triethyl amine.

**In** a preferred embodiment, the molar ratio of the alkaline compound to the lysine and/or lysine salt is (1.0-2.0) :1. The alkaline compound can convert the lysine or lysine salt into a sodium salt or potassium salt, making it easily soluble in water. The alkaline compound can also act as an acid binding agent, to promote the progress of the reaction.

**In** a preferred embodiment, the raw materials include an emulsifying agent. In a preferred embodiment, the emulsifying agent includes at least one of sodium lauryl sulfate, sodium oleate, polyoxyethylene cetyl ether, and polyoxyethylene monooleate. In a preferred embodiment, the amount of the emulsifying agent is 20% by weight of the lysine and/or lysine salt. The emulsifying agent improves the stability of the emulsion, and can be optionally added and used according to actual needs.

In a preferred embodiment, the lauroyl halide has a structure of an expression formula below: 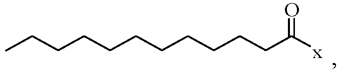 where X is halo that is specifically fluoro, chloro, or bromo. The lauroyl halide is most preferably lauroyl chloride.

In a preferred embodiment, the molar ratio of the lysine and/or lysine salt to the lauric acid and/or lauric acid derivative is 2:1 to 1:2.

In a preferred embodiment, the reaction process is as follows. At a temperature of -5 to 50°C, the alkaline compound is added to the two-phase microemulsion to control the pH in the range of 9 to 14. Lauric acid and/or a lauroyl halide are added dropwise to the two-phase microemulsion for reaction over a time controlled within 1 to 2 hrs, and then the reaction is continuously stirred for 0.5 to 12 hrs. During the reaction, the alkaline compound is added to control the pH of the system. The alkaline compound is at least one of sodium hydroxide, potassium hydroxide, and triethyl amine. The reaction temperature is further preferably -5 to 10°C. As the temperature increases, the stability of the two-phase microemulsion will become worse, and the side reactions will increase. For example, the hydrolysis product of the lauroyl halide will increase with the increase of the reaction temperature.

In a preferred embodiment, the lysine salt includes lysine hydrochloride, lysine sulfate, sodium lysine, potassium lysine and the like.

In a preferred embodiment, hydrochloric acid, sulfuric acid, acetic acid or the like, and preferably hydrochloric acid, is used as a neutralization reagent in the neutralization reaction. During the neutralization reaction, the pH of the solution is controlled in the range of 3 to 8.

### Beneficial effects

Compared with the prior art, the technical solution of the present invention has the following beneficial technical effects.

According to the technical solution of the present invention, N^{ε}-lauroyl lysine is obtained with high selectivity by using a "two-phase method", which greatly reduces the production of by-products such as N^{α}-lauroyl lysine. Moreover, the method does not need protection with a group or chelation protection with a metal ion. The product can be synthesized by a one-step reaction, the reaction selectivity is high, the operation is simple, the reaction conditions are mild, the separation and purification of the product are simple, and the yield is high. Thus, the method is suitable for use in industrial production.

### BRIEF DESCRIPTION OF THE DRAWINGS

Fig. 1 shows a blank control;
Fig. 2 shows the liquid chromatographic analysis of N^{α}-lauroyl lysine;
Fig. 3 shows the liquid chromatographic analysis of a N^{α}/N^{ε}-lauroyl lysine mixture prepared in Comparative Example 2;
Fig. 4 shows N^{ε}-lauroyl lysine (a competitive product from other countries); and
Fig. 5 shows the liquid chromatographic analysis of N^{ε}-lauroyl lysine prepared in Example 1.

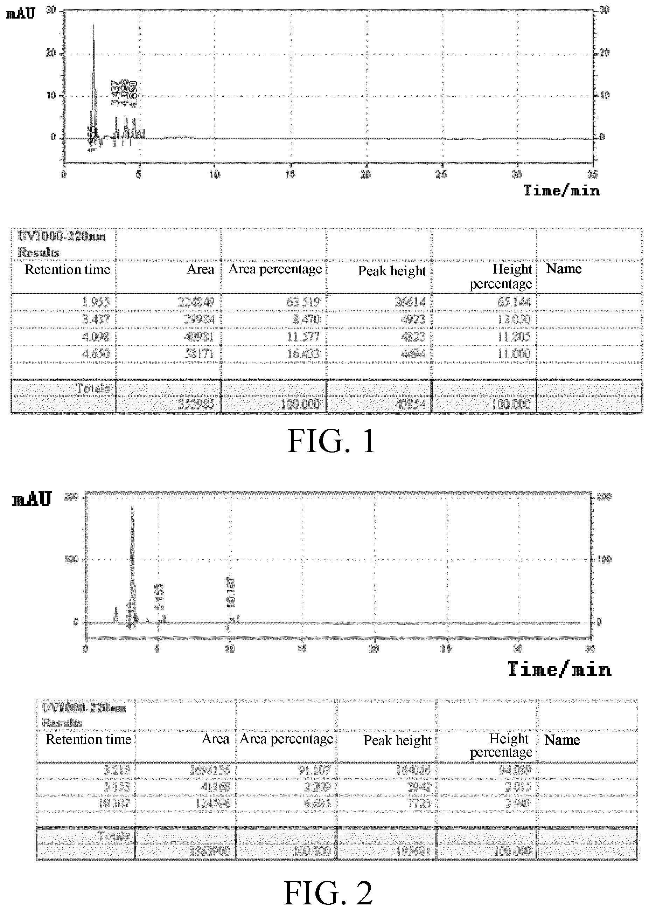

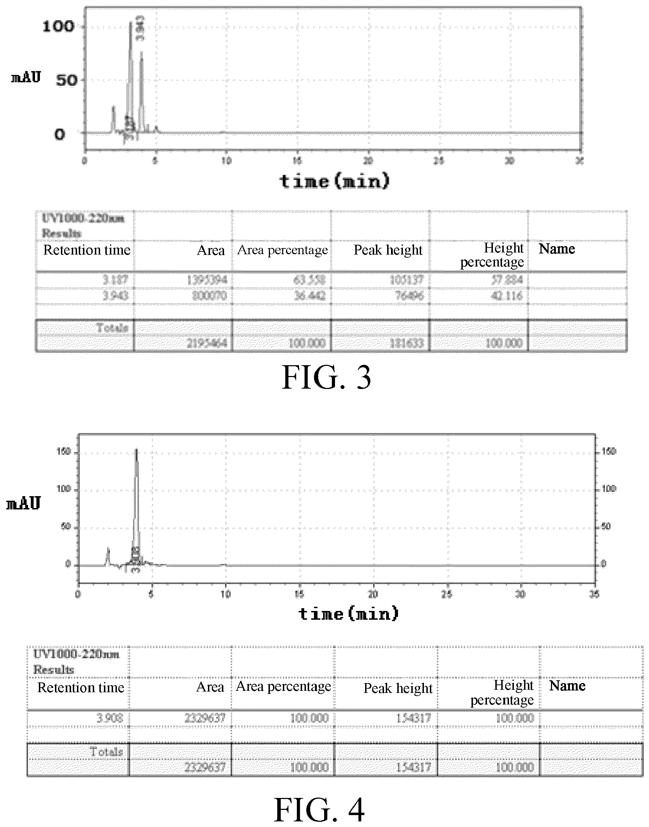

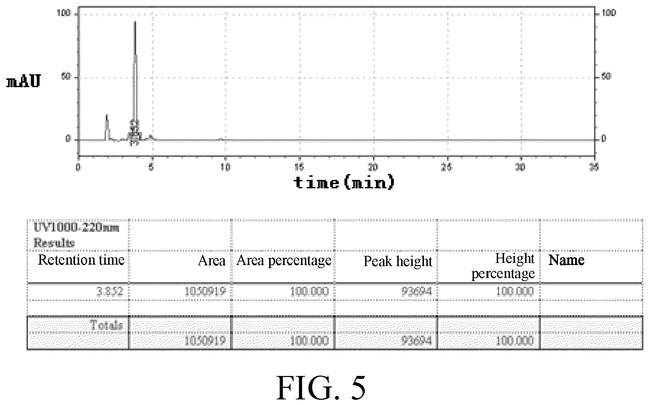

Figs. 1 to 5 show that a high-purity N^{ε}-lauroyl lysine product is prepared in Example 1.

### DETAILED DESCRIPTION

Hereinafter, the present invention is further described in detail with reference to specific examples. However, the protection scope of the claims is not limited thereto.

### Example 1

11.0 g of lysine hydrochloride, 4.0 g of sodium hydroxide, and 12.0 g of water were added into a 500 mL four-neck round-bottom flask equipped with a stirrer, and dissolved by stirring at room temperature. Then, 40 mL of ethanol, 80 mL of n-hexane and 0.2 g of sodium lauryl sulfate (emulsifying agent) were added and stirred until uniform. A transparent microemulsion was formed after about 30 min. The reaction solution was cooled to 10°C. 12.5 g of lauroyl chloride (0.95 equiv.) was slowly added dropwise, during which a 30% aqueous sodium hydroxide solution was also added dropwise to maintain the pH at 11. The process was continued for about 1.5 hrs, and the reaction was stirred for another 1 hr after addition. After the reaction, the reaction solution was returned to room temperature. 6 M hydrochloric acid was slowly added dropwise to adjust the pH to about 6. The reaction solution was transferred to a separatory funnel, and allowed to stand. The lower aqueous phase was separated and removed. After filtration under suction, a white solid product was obtained, which was washed with a small amount of ethanol/water solution (2:1 volume ratio), and dried in an oven, to obtain 14.2 g of the target product N^{ε}-lauroyl lysine as a white powder with a yield of 76% and a HPLC purity of higher than 97%.

### Example 2

11.0 g of lysine hydrochloride, 4.0 g of sodium hydroxide, and 12.0 g of water were added into a 500 mL four-neck round-bottom flask equipped with a stirrer, and dissolved by stirring at room temperature. Then, 40 mL of ethanol, 80 mL of n-hexane and 0.2 g of sodium lauryl sulfate (emulsifying agent) were added and stirred until uniform. A transparent microemulsion was formed after about 30 min. The reaction solution was cooled to 10°C. 13.8 g of lauroyl chloride (1.05 equiv.) was slowly added dropwise, during which a 30% aqueous sodium hydroxide solution was also added dropwise to maintain the pH at 11. The process was continued for about 1.5 hrs, and the reaction was stirred for another 1 hr after addition. After the reaction, the reaction solution was returned to room temperature. 6 M hydrochloric acid was slowly added dropwise to adjust the pH to about 6. The reaction solution was transferred to a separatory funnel, and allowed to stand. The lower aqueous phase was separated and removed. After filtration under suction, a white solid product was obtained, which was washed with a small amount of ethanol/water solution (2:1 volume ratio), and dried in an oven, to obtain 13.4 g of the target product N^{ε}-lauroyl lysine as a white powder with a yield of 68% and a HPLC purity of 94%.

### Example 3

11.0 g of lysine hydrochloride, 4.0 g of sodium hydroxide, and 12.0 g of water were added into a 500 mL four-neck round-bottom flask equipped with a stirrer, and dissolved by stirring at room temperature. Then, 40 mL of ethanol, 80 mL of n-hexane and 0.2 g of sodium lauryl sulfate (emulsifying agent) were added and stirred until uniform. A transparent microemulsion was formed after about 30 min. The reaction solution was cooled to 10°C. 15.8 g of lauroyl chloride (1.2 equiv.) was slowly added dropwise, during which a 30% aqueous sodium hydroxide solution was also added dropwise to maintain the pH at 11. The process was continued for about 1.5 hrs, and the reaction was stirred for another 1 hr after addition. After the reaction, the reaction solution was returned to room temperature. 6 M hydrochloric acid was slowly added dropwise to adjust the pH to about 6. The reaction solution was transferred to a separatory funnel, and allowed to stand. The lower aqueous phase was separated and removed. After filtration under suction, a white solid product was obtained, which was washed with a small amount of ethanol/water solution (2:1 volume ratio), and dried in an oven, to obtain 13.8 g of the target product N^{ε}-lauroyl lysine as a white powder with a yield of 70% and a HPLC purity of 92%.

### Example 4

11.0 g of lysine hydrochloride, 4.0 g of sodium hydroxide, and 12.0 g of water were added into a 500 mL four-neck round-bottom flask equipped with a stirrer, and dissolved by stirring at room temperature. Then, 50 mL of ethanol, 80 mL of n-hexane and 0.2 g of polyoxyethylene cetyl ether (emulsifying agent) were added and stirred until uniform. A transparent microemulsion was formed after about 30 min. The reaction solution was cooled to 10°C. 12.5 g of lauroyl chloride (0.95 equiv.) was slowly added dropwise, during which a 30% aqueous sodium hydroxide solution was also added dropwise to maintain the pH at 11. The process was continued for about 1.5 hrs, and the reaction was stirred for another 1 hr after addition. After the reaction, the reaction solution was returned to room temperature. 6 M hydrochloric acid was slowly added dropwise to adjust the pH to about 6. The reaction solution was transferred to a separatory funnel, and allowed to stand. The lower aqueous phase was separated and removed. After filtration under suction, a white solid product was obtained, which was washed with a small amount of ethanol/water solution (2:1 volume ratio), and dried in an oven, to obtain 13.1g of the target product N^{ε}-lauroyl lysine as a white powder with a yield of 70% and a HPLC purity of higher than 97%.

### Example 5

11.0 g of lysine hydrochloride, 4.0 g of sodium hydroxide, and 12.0 g of water were added into a 500 mL four-neck round-bottom flask equipped with a stirrer, and dissolved by stirring at room temperature. Then, 30 mL of ethanol and 80 mL of n-hexane (without an emulsifying agent) were added and stirred until uniform. A transparent microemulsion was formed after about 30 min. The reaction solution was cooled to 10°C. 12.5g of lauroyl chloride (0.95 equiv.) was slowly added dropwise, during which a 30% aqueous sodium hydroxide solution was also added dropwise to maintain the pH at 11. The process was continued for about 1.5 hrs, and the reaction was stirred for another 1 hr after addition. After the reaction, the reaction solution was returned to room temperature. 6 M hydrochloric acid was slowly added dropwise to adjust the pH to about 6. The reaction solution was transferred to a separatory funnel, and allowed to stand. The lower aqueous phase was separated and removed. After filtration under suction, a white solid product was obtained, which was washed with a small amount of ethanol/water solution (2:1 volume ratio), and dried in an oven, to obtain 11.6 g of the target product N^{ε}-lauroyl lysine as a white powder with a yield of 62% and a HPLC purity of higher than 97%.

### Example 6

11.0 g of lysine hydrochloride, 4.0 g of potassium hydroxide, and 12.0 g of water were added into a 500 mL four-neck round-bottom flask equipped with a stirrer, and dissolved by stirring at room temperature. Then, 40 mL of ethanol, 80 mL of n-hexane and 0.2 g of sodium lauryl sulfate (emulsifying agent) were added and stirred until uniform. A transparent microemulsion was formed after about 30 min. At room temperature, 12.5 g of lauroyl chloride (0.95 equiv.) was slowly added dropwise, during which a 30% aqueous sodium hydroxide solution was also added dropwise to maintain the pH at 12. The process was continued for about 1.5 hrs, and the reaction was stirred for another 1 hr after addition. After the reaction, the reaction solution was returned to room temperature. 6 M hydrochloric acid was slowly added dropwise to adjust the pH to about 6. The reaction solution was transferred to a separatory funnel, and allowed to stand. The lower aqueous phase was separated and removed. After filtration under suction, a white solid product was obtained, which was washed with a small amount of ethanol/water solution (2:1 volume ratio), and dried in an oven, to obtain 13.3 g of the target product N^{ε}-lauroyl lysine as a white powder with a yield of 71% and a HPLC purity of 92%.

### Example 7

11.0 g of lysine hydrochloride, 4.0 g of sodium hydroxide, and 12.0 g of water were added into a 500 mL four-neck round-bottom flask equipped with a stirrer, and dissolved by stirring at room temperature. Then, 40 mL of methanol, 80 mL of n-hexane and 0.2 g of sodium lauryl sulfate (emulsifying agent) were added and stirred until uniform. A transparent microemulsion was formed after about 30 min. At room temperature, 12.5 g of lauroyl chloride (0.95 equiv.) was slowly added dropwise, during which a 30% aqueous sodium hydroxide solution was also added dropwise to maintain the pH at 11. The process was continued for about 1.5 hrs, and the reaction was stirred for another 1 hr after addition. After the reaction, 6 M hydrochloric acid was slowly added dropwise to adjust the pH to about 6 at room temperature. The reaction solution was transferred to a separatory funnel, and allowed to stand. The lower aqueous phase was separated and removed. After filtration under suction, a white solid product was obtained, which was washed with a small amount of ethanol/water solution (2:1 volume ratio), and dried in an oven, to obtain 10.3 g of the target product N^{ε}-lauroyl lysine as a white powder with a yield of 55% and a HPLC purity of higher than 97%.

### Example 8

11.0 g of lysine hydrochloride, 4.0 g of sodium hydroxide, and 12.0 g of water were added into a 500 mL four-neck round-bottom flask equipped with a stirrer, and dissolved by stirring at room temperature. Then, 40 mL of isopropanol, 80 mL of n-hexane and 0.2 g of sodium lauryl sulfate (emulsifying agent) were added and stirred until uniform. A transparent microemulsion was formed after about 30 min. At room temperature, 12.5 g of lauroyl chloride (0.95 equiv.) was slowly added dropwise, during which a 30% aqueous sodium hydroxide solution was also added dropwise to maintain the pH at 11. The process was continued for about 1.5 hrs, and the reaction was stirred for another 1 hr after addition. After the reaction, 6 M hydrochloric acid was slowly added dropwise to adjust the pH to about 6 at room temperature. The reaction solution was transferred to a separatory funnel, and allowed to stand. The lower aqueous phase was separated and removed. After filtration under suction, a white solid product was obtained, which was washed with a small amount of ethanol/water solution (2:1 volume ratio), and dried in an oven, to obtain 7.8 g of the target product N^{ε}-lauroyl lysine as a white powder with a yield of 42% and a HPLC purity of higher than 97%.

### Example 9

11.0 g of lysine hydrochloride, 4.0 g of sodium hydroxide, and 12.0 g of water were added into a 500 mL four-neck round-bottom flask equipped with a stirrer, and dissolved by stirring at room temperature. Then, 40 mL of ethanol, 80 mL of petroleum ether and 0.2 g of sodium lauryl sulfate (emulsifying agent) were added and stirred until uniform. A transparent microemulsion was formed after about 30 min. The reaction solution was cooled to 10°C. 12.5 g of lauroyl chloride (0.95 equiv.) was slowly added dropwise, during which a 30% aqueous sodium hydroxide solution was also added dropwise to maintain the pH at 11. The process was continued for about 1.5 hrs, and the reaction was stirred for another 1 hr after addition. After the reaction, the reaction solution was returned to room temperature. 6 M hydrochloric acid was slowly added dropwise to adjust the pH to about 6. The reaction solution was transferred to a separatory funnel, and allowed to stand. The lower aqueous phase was separated and removed. After filtration under suction, a white solid product was obtained, which was washed with a small amount of ethanol/water solution (2:1 volume ratio), and dried in an oven, to obtain 13.8 g of the target product N^{ε}-lauroyl lysine as a white powder with a yield of 74% and a HPLC purity of higher than 97%.

### Example 10

11.0 g of lysine hydrochloride, 4.0 g of sodium hydroxide, and 12.0 g of water were added into a 500 mL four-neck round-bottom flask equipped with a stirrer, and dissolved by stirring at room temperature. Then, 40 mL of ethanol, 60 mL of n-hexane and 0.2 g of sodium lauryl sulfate (emulsifying agent) were added and stirred until uniform. A transparent microemulsion was formed after about 30 min. The reaction solution was cooled to 10°C. 12.5 g of lauroyl chloride (0.95 equiv.) was slowly added dropwise, during which a 30% aqueous sodium hydroxide solution was also added dropwise to maintain the pH at 10. The process was continued for about 1.5 hrs, and the reaction was stirred for another 1 hr after addition. After the reaction, the reaction solution was returned to room temperature. 6 M hydrochloric acid was slowly added dropwise to adjust the pH to about 6. The reaction solution was transferred to a separatory funnel, and allowed to stand. The lower aqueous phase was separated and removed. After filtration under suction, a white solid product was obtained, which was washed with a small amount of ethanol/water solution (2:1 volume ratio), and dried in an oven, to obtain 12.42 g of the target product N^{ε}-lauroyl lysine as a white powder with a yield of 66% and a HPLC purity of higher than 97%.

### Example 11

11.0 g of lysine hydrochloride, 4.0 g of sodium hydroxide, and 12.0 g of water were added into a 500 mL four-neck round-bottom flask equipped with a stirrer, and dissolved by stirring at room temperature. Then, 40 mL of ethanol, 40 mL of n-hexane and 0.2 g of sodium lauryl sulfate (emulsifying agent) were added and stirred until uniform. A transparent microemulsion was formed after about 30 min. The reaction solution was cooled to 10°C. 12.5 g of lauroyl chloride (0.95 equiv.) was slowly added dropwise, during which a 30% aqueous sodium hydroxide solution was also added dropwise to maintain the pH at 11. The process was continued for about 1.5 hrs, and the reaction was stirred for another 1 hr after addition. After the reaction, the reaction solution was returned to room temperature. 6 M hydrochloric acid was slowly added dropwise to adjust the pH to about 6. The reaction solution was transferred to a separatory funnel, and allowed to stand. The lower aqueous phase was separated and removed. After filtration under suction, a white solid product was obtained, which was washed with a small amount of ethanol/water solution (2:1 volume ratio), and dried in an oven, to obtain 10.1 g of the target product N^{ε}-lauroyl lysine as a white powder with a yield of 54% and a HPLC purity of higher than 97%.

### Example 12

11.0 g of lysine hydrochloride, 4.0 g of sodium hydroxide, and 12.0 g of water were added into a 500 mL four-neck round-bottom flask equipped with a stirrer, and dissolved by stirring at room temperature. Then, 20 mL of ethanol, 80 mL of n-hexane and 0.2 g of sodium lauryl sulfate (emulsifying agent) were added and stirred until uniform. A transparent microemulsion was formed after about 30 min. The reaction solution was cooled to 10°C. 12.5 g of lauroyl chloride (0.95 equiv.) was slowly added dropwise, during which a 30% aqueous sodium hydroxide solution was also added dropwise to maintain the pH at 11. The process was continued for about 1.5 hrs, and the reaction was stirred for another 1 hr after addition. After the reaction, the reaction solution was returned to room temperature. 6 M hydrochloric acid was slowly added dropwise to adjust the pH to about 6. The reaction solution was transferred to a separatory funnel, and allowed to stand. The lower aqueous phase was separated and removed. After filtration under suction, a white solid product was obtained, which was washed with a small amount of ethanol/water solution (2:1 volume ratio), and dried in an oven, to obtain 11.6 g of the target product N^{ε}-lauroyl lysine as a white powder with a yield of 62% and a HPLC purity of higher than 97%.

### Comparative Example 1

11.0 g of lysine hydrochloride, 4.0 g of sodium hydroxide, and 12.0 g of water were added into a 500 mL four-neck round-bottom flask equipped with a stirrer, and dissolved by stirring at room temperature. 80 mL of n-hexane and 0.2 g of sodium lauryl sulfate (emulsifying agent) were then added and stirred until uniform. The reaction solution was cooled to 10°C. 12.5 g of lauroyl chloride (0.95 equiv.) was slowly added dropwise, during which a 30% aqueous sodium hydroxide solution was also added dropwise to maintain the pH at about 11. The process was continued for about 1.5 hrs, and the reaction was stirred for another 1 hr after addition. After the reaction, the reaction solution was returned to room temperature. 6 M hydrochloric acid was slowly added dropwise to adjust the pH to about 6. The reaction solution was transferred to a separatory funnel, and allowed to stand. The lower aqueous phase was separated and removed. After filtration under suction, a white solid product was obtained. The product was sticky and difficult to be dried under suction. The product was simply recrystallized in ethanol/water (2:1 volume ratio), filtered suction, washed with a small amount of ethanol/water solution (2:1 volume ratio), and dried in an oven, to obtain 3.0 g of the target product N^{ε}-lauroyl lysine as a white powder with a yield of 16% and a HPLC purity of greater than 97%.

### Comparative Example 2

11.0 g of lysine hydrochloride, 4.0 g of sodium hydroxide, and 12.0 g of water were added into a 500 mL four-neck round-bottom flask equipped with a stirrer, and dissolved by stirring at room temperature. Then, 40 mL of ethanol was added and stirred until uniform. The reaction solution was cooled to 10°C. 12.5 g of lauroyl chloride (0.95 equiv.) was slowly added dropwise, during which a 30% aqueous sodium hydroxide solution was also added dropwise to maintain the pH at about 11. The process was continued for about 1.5 hrs, and the reaction was stirred for another 1 hr after addition. After the reaction, the reaction solution was returned to room temperature. 6 M hydrochloric acid was slowly added dropwise to adjust the pH to about 6. After filtration under suction, a white solid product was obtained. The product was sticky and difficult to be dried under suction. The product was simply recrystallized in ethanol/water (2:1 volume ratio), filtered suction, washed with a small amount of ethanol/water solution (2:1 volume ratio), and dried in an oven, to obtain 6.1 g of the target product N^{ε}-lauroyl lysine as a white powder with a yield of 33% and a HPLC purity of 36.44%.

To sum up, preferred specific embodiments of the present invention are described, and the protection scope of the present invention is not limited thereto. Any simple changes or equivalent replacements made by any person of skill in the art within the technical scope disclosed in the present invention to the technical solution are embraced in the protection scope of the present invention.

## Claims

1. A method for synthesizing high-purity N^{ε}-lauroyl lysine, comprising the following steps:
1) stirring raw materials including lysine and/or a lysine salt, an alkaline compound, water, a lower alcohol, and a water-insoluble solvent until uniform to form a two-phase microemulsion; and
2) adding lauric acid and/or lauroyl halide dropwise to the two-phase microemulsion, reacting with stirring, and precipitating by neutralization with an acid, to obtain N^{ε}-lauroyl lysine.

2. The method for synthesizing high-purity N^{ε}-lauroyl lysine according to claim 1, wherein the ratio of the total volume of the water and the lower alcohol to the volume of the water-insoluble solvent is 1: 1 to 1:5.

3. The method for synthesizing high-purity N^{ε}-lauroyl lysine according to claim 2, wherein the volume ratio of the water and the lower alcohol is (5-20):40.

4. The method for synthesizing high-purity N^{ε}-lauroyl lysine according to any one of claims 1 to 3, wherein the lower alcohol is a C₁-C₄ alcohol.

5. The method for synthesizing high-purity N^{ε}-lauroyl lysine according to claim 1 or 2, wherein the water-insoluble solvent comprises at least one of petroleum ether, n-hexane, cyclohexane, toluene, and dichloromethane.

6. The method for synthesizing high-purity N^{ε}-lauroyl lysine according to claim 1, wherein
the alkaline compound comprises at least one of sodium hydroxide, potassium hydroxide, and triethyl amine; and
the molar ratio of the alkaline compound to the lysine and/or lysine salt is (1.0-2.0) :1.

7. The method for synthesizing high-purity N^{ε}-lauroyl lysine according to claim 1, wherein
the raw materials comprises an emulsifying agent; and
the emulsifying agent comprises at least one of sodium lauryl sulfate, sodium oleate, polyoxyethylene cetyl ether, and polyoxyethylene monooleate.

8. The method for synthesizing high-purity N^{ε}-lauroyl lysine according to claim 7, wherein the amount of the emulsifying agent is 20% or less by weight of the lysine and/or lysine salt.

9. The method for synthesizing high-purity N^{ε}-lauroyl lysine according to claim 1, wherein the molar ratio of the lysine and/or lysine salt to the lauric acid and/or lauroyl halide is 2:1-1:2.

10. The method for synthesizing high-purity N^{ε}-lauroyl lysine according to claim 1, wherein the reaction process comprises adding the alkaline compound to the two-phase microemulsion at a temperature of -5 to 50°C, to control the pH in the range of 9 to 14; adding lauric acid and/or a lauroyl halide dropwise to the two-phase microemulsion for reaction over a time controlled within 1 to 2 hrs; and then continuously stirring the reaction for 0.5 to 12 hrs.
